# EUROPEAN PATENT SPECIFICATION

(11) **EP 1 234 544 B1**
(45) Date of publication and mention of the grant of the patent: **12.09.2007**
(21) Application number: 02450038.1
(22) Date of filing: 27.02.2002
(51) Int. Cl.: A61B 17/04

(54) **Insert molded push-in suture anchor**
Eingegossener Einstecknähfadenanker
Dispositif d'ancrage de suture à emboîtement moulé par insertion

(30) Priority: 27.02.2001 US 271414 P
(43) Date of publication of application: 28.08.2002
(73) Proprietor: Arthrex Inc, Naples, Florida 34104 (US)
(72) Inventor: Benavitz, William C., Naples, Florida 34109 (US); Grafton, Donald R., Naples, Florida 34104 (US); Schmieding, Reinhold, Naples, Florida 34108 (US)
(74) Representative: Kopecky, Helmut

(56) References cited:
- EP-A- 0 632 999
- US-A- 5 417 712
- US-A- 5 578 057
- US-A- 5 964 783
- US-A- 6 117 162

## Description

### BACKGROUND OF THE INVENTION

### 1. Field of the Invention:

The present invention relates to an apparatus for anchoring surgical suture to bone. More specifically, the present invention relates to arthroscopic apparatus and methods for anchoring suture to bone using a push-in suture anchor having suture molded directly into the body of the suture anchor.

### 2. Description of the Related Art:

When soft tissue tears away from bone, reattachment becomes necessary. Various fixation devices, including sutures, screws, staples, wedges, and plugs have been used in the past to secure soft tissue to bone. More recently, various types of threaded suture anchors have been developed.

Suture anchors and implants generally include a structure for attaching or securing the suture to the anchor. U.S. Patent No. 4,632,100, for example, discloses and claims a threaded suture anchor with a complex press-fitted disc and knot structure which secures the suture to the anchor. In other suture anchors, such as those disclosed in U.S. Patent No. 5,370,662, the suture is attached to the anchor by passing the suture through an eyelet at the back end of the anchor. Problems can arise if the structure for attaching the suture fails, allowing the suture to become detached from the anchor. Also, in some of the known devices, the suture is exposed to abrasion or cutting by sharp or rough areas along the walls of the bone canal into which the anchor is inserted

EP A-0 632 999 (D1) discloses a suture anchor formed of a plurality of truncated cones (Fig. 2), but fails to disclose that the anchor terminates at its distal end in a truncated cone with a blunt tip. D1 also fails to disclose a round and tapered drive head at the proximal end of the suture anchor. D1 also fails to disclose a suture insert-molded into the anchor in the form of a suture loop which extends outside the anchor body at the proximal end of the anchor to provide a suture eyelet.

US 5,964,783 (D2) relates to an insert-molded suture anchor having a body molded around a loop of suture which extends outside the Suture body at the proximal end to provide a suture eyelet. However, the anchor of D2 has a threaded configuration, and is not formed of a plurality of truncated cones, terminating at its distal end in a blunt tip. D2 also fails to disclose a round and tapered drive head at the proximal end of the suture anchor, or a hand driver (claim 3) with an open round recess for receiving the round and tapered proximal end of the anchor.

US 5,578,057 (D3) teaches an installation tool for deploying an anchoring device in a preformed hole, the anchoring device being attached to a suture carrying a needle. The installation tool of D3 is provided with (1) positioning means for engaging an anchor and locating the anchor at a desired position within a hole, and (2) retention means releasably associated with the positioning means. D3 does not disclose a driver with an open round recess for receiving the round and tapered proximal end of a suture anchor as recited in claim 3.

US 6,117,162 (D4) relates to a metal suture anchor having a threaded configuration with the thread spiralling around a tapering central core. Although the threaded suture anchor of D4 may be employed with a hand driver, the hand driver does not have an open round recess for receiving the round and tapered proximal end of a suture anchor as recited in claim 3. Rather, the head of the driver is provided in the shape of a hexagonal socket to match the shape of the drive head on the anchor, so that the anchor can be turned into bone. The suture anchor of D4 does not have a body formed of plurality of truncated cones, terminating at its distal end in a truncated cone with a blunt tip. The drive head of the anchor is not round and tapered, but rather is polygonal in shape so that the anchor can be turned into bone (rather than pushed into bone) with a corresponding shaped driver. D4 also fails to disclose a suture insert-molded into the anchor in the form of a suture loop which extends outside the anchor body at the proximal end of the anchor to provide a suture eyelet.

US 5,417,712 (D5) teaches an anchoring device 3 having bone-engaging means 45 that are cantilevered to curved central portion 42 and formed of a shape memory alloy, and not an insert-molded suture anchor, much less an insert-molded suture anchor. The suture anchor of D5 does not have a body formed of a plurality of truncated cones, terminating at its distal end in a truncated cone with a blunt tip. D5 also fails to disclose a round and tapered drive head at the proximal end of the suture anchor. D51 also fails to disclose a suture insert-molded into the anchor in the form of a suture loop which extends outside the anchor body at the proximal end of the anchor to provide a suture eyelet. having a suture loop extending outside the anchor body at the proximal end of the anchor and a plurality of adjacent truncated cones.

In addition, the eyelet or, in the case of U.S. Patent No. 4,632,100, the axial opening for receiving the disc to which the suture is knotted, is formed as part of the drive head of the known suture anchors, which weakens the drive head. Various other modifications in the drive head are often employed in connection with suture attachment. For example, recessed grooves may be formed on opposite sides of the drive head to receive and protect the suture from the abrasive areas of the suture anchor tunnel. In such cases, the drive head often is made of a larger diameter to recover the mechanical strength lost from the removal of material relating to the suture-attachment or suture-protection modification.

Accordingly, a need exists for a suture anchor or implant to which suture is secured effectively so as to prevent detachment of the suture, A need also exists for a soft tissue fixation device having a low profile configuration particularly suited for reattachment of tissue to the glenoid rim, for example.

### SUMMARY OF THE INVENTION

The suture anchor of the present invention overcomes disadvantages of the prior art, such as those noted above, and achieves the foregoing objectives by providing a push in suture anchor having suture insert-molded into the suture anchor during the manufacturing process.

The insert-molded suture anchor formed by placing a suture in a mold and molding a suture anchor body around the suture according to the present invention comprises an anchor body comprising a plurality of adjacent truncated cones, the anchor body terminating at its distal end in a truncated cone with a blunt tip; a round and tapered drive head at the proximal end of the anchor body; and a suture, insert-molded with the anchor body in the form of a suture loop, the suture loop extending outside the anchor body at the proximal end of the anchor to provide a suture eyelet.

Advantageously, the suture exits the suture anchor along the central axis of the anchor, which prevents suture abrasion by the wall of the bone tunnel into which the anchor is inserted.

The suture insert-molded within the anchor body according to an embodiment of the invention advantageously extends through the entire length of the anchor.

The insert-molded suture anchor is advantageously combined with a hand driver having a cannulated shaft with an open round recess on an end of the shaft for receiving the round and tapered proximal end of the suture anchor to allow an easy placement of the suture anchor.

The suture anchor according to the present invention is formed by a process comprising the steps of placing at least one piece of suture in a mold; molding a polymer around the suture by delivering an uncured polymer into the mold, and causing the polymer to cure, to form an insert-molded suture anchor.

Other features and advantages of the present invention will become apparent from the following description of the invention, which refers to the accompanying

### BRIEF DESCRIPTION OF THE DRAWINGS

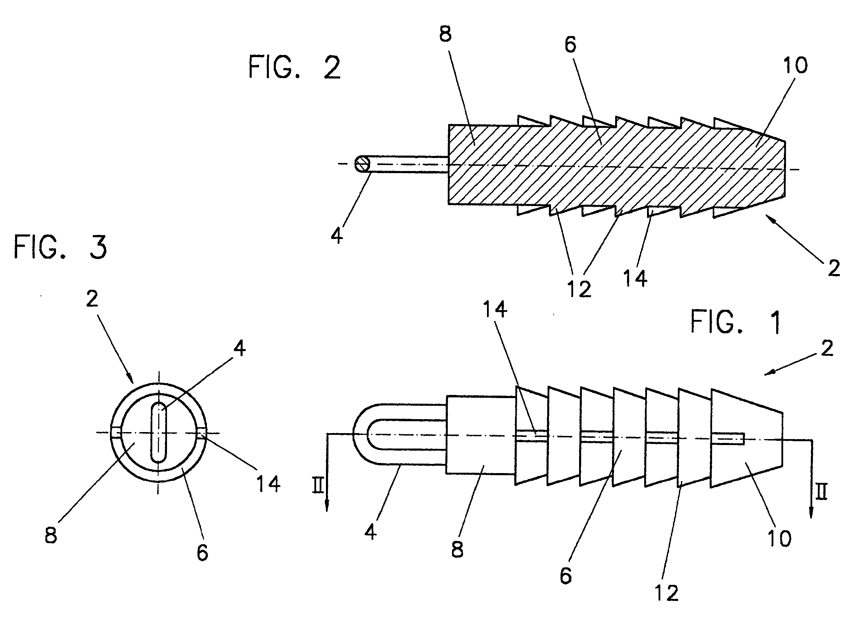

Fig. 1 is a plan view of the push-in suture anchor of the present invention.
Fig. 2 is a sectional elevation of the suture anchor of Fig. 1.
Fig. 3 is a proximal end view of the suture anchor of Fig. 1.
Fig. 4 is a plan view of a hand driver for inserting the push-in suture anchor of the present invention.
Fig. 5 is an elevation view of the hand driver of Fig. 4.
Fig. 6 is a sectional view of the hand driver of Fig. 4.
Fig. 7 is a detail view of the drive end of the hand driver of Fig. 4.
Fig. 8 is a plan view of an alternative hand driver for a method of capsular plication using the push-in suture anchor according to the present invention.
Fig. 9 is an elevational view of the hand driver of Fig. 8.
Fig. 10 is a sectional elevation of the hand driver of Fig. 8.
Fig. 11 is a detail view of the drive end of the hand driver of Fig. 4.

### DETAILED DESCRIPTION OF THE PREFERRED EMBODIMENTS

Referring to Pigs. 1-3, the present invention is shown as a push-in suture anchor **2** having suture **4** that is insert-molded directly into the suture anchor body **6** during the manufacturing process.

The suture anchor body **6** preferably is formed of a bioabsorbable material, poly(l-lactide-co-d,l-lactide) 70:30 (PLDLA) being most preferred. Suture **4** can be any known type of suture selected according to the size of the anchor and the anticipated application. The suture **4** preferably is No. 2 polyester braided suture.

At least one length of the insert-molded suture **4** extends from the proximal end of the suture anchor body. Preferably, the suture extends from the suture anchor body in the form of a loop. Various methods of increasing the pull out strength of the suture from the anchor body are known from the state of the art.

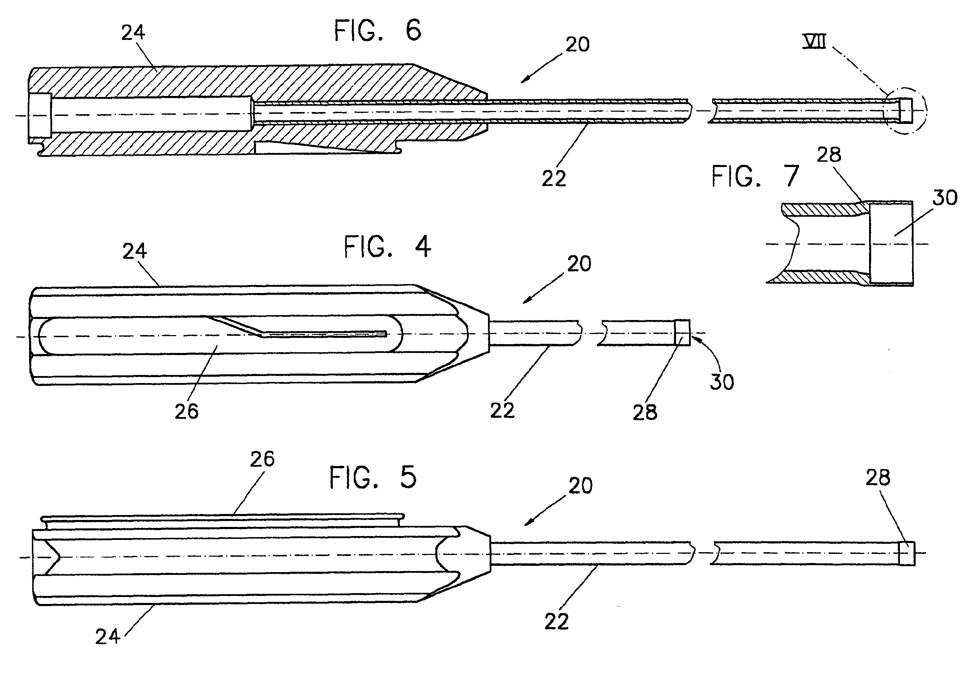

The proximal end **8** of the suture anchor body is tapered for a snug fit into a hand driver described below, for example, with reference to Figs. 4-7. The distal end **10** of the suture anchor tapers to a blunt tip. Suture anchor **2** is provided with slotted ribs **12** formed circumferentially at least partially around and partially along the length of body **6.** Ribs **12** have a truncated, conical shape, each rib increasing in diameter toward the head of the anchor at an angle of preferably 15° with respect to the longitudinal axis of anchor **2**, and reaching a major diameter of 3.0 mm. Slots **14** are formed in ribs **12** on alternating sides of body 4.

Referring to Figs. 4-7, a hand driver **20** according to the present invention is shown. Hand driver **20** includes a cannulated shaft **22** with a cannulated handle **24.** A cleat **26** is provided on the handle for securing suture attached to the suture loop on the suture anchor and passed through the cannulated shaft and handle. The distal tip **28** of cannulated shaft **22** provides a recess **30** which receives the proximal end of suture anchor **2.** The outer diameter of the distal end of the driver preferably is less than or equal to the maximum outer diameter of the suture anchor.

The suture anchor is pushed into a hole formed in bone. The hole can be formed by punching or boring, for example. The ribs secure the anchor in the bone. The slots enhance attachment in the bone and support bony in-growth for increased pull out strength.

Advantageously, the hole formed in bone is made deep enough, and the suture anchor is advanced into the hole sufficiently, so that the proximal end of the anchor sits flush with or below the bone surface. Accordingly, the repair leaves a smooth bone surface, minimizing or eliminating abrasion or other damage to surrounding soft tissue. The anchor generally becomes encapsulated by fibrous tissue within six weeks after implantation.

Although PLDLA is the most preferred material for the suture anchor of the present invention, other bioabsorbable materials known in the art can be utilized. As used herein, bioabsorbable is considered to be interchangeable with biodegradable, resorbable and absorbable to mean that the device can be at least partially absorbed by the body over time. Preferably, the anchor material is selected so as to absorb or degrade substantially completely within 12-16 months of implantation.

The push-in suture anchor of the present invention is particularly well suited for reattachment of the glenoid labrum or inferior glenohumeral ligament in patients with primary or recurrent anterior dislocation or subluxation of the shoulder in association with adequate post-operative immobilization. More specifically, the anchor also can be used for repair procedures such as capsulabral plication, as described below.

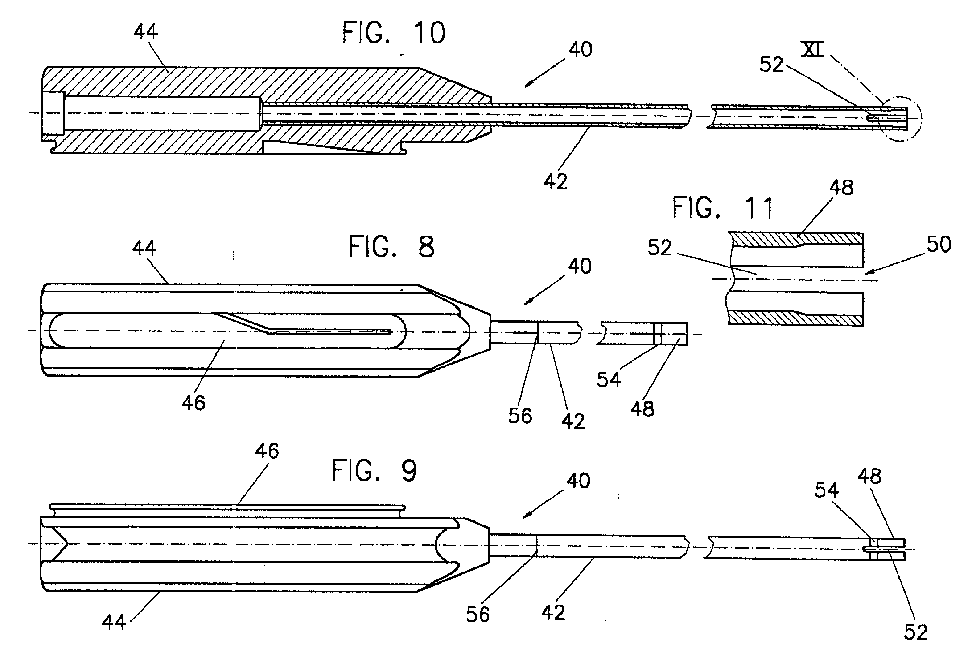

Referring to Figs. 8-11, a driver **40** for capsule plication using the anchor according to the present invention is shown. Capsulolabral plication is indicated for repair of certain types of shoulder laxity. When pathologically increased anterior laxity is combined with a Bankart lesion, for example, the addition of a capsular plication to the reattachment of the capsulolabral avulsion has been recommended.

Driver **40** includes a cannulated shaft **42** with a cannulated handle **44**. A cleat **46** is provided on the handle for securing suture attached to the suture loop on the suture anchor and passed through the cannulated shaft and handle. The distal tip **48** of cannulated shaft **42** provides a recess **50** which receives the proximal end of suture anchor **2**. The outer diameter of the distal end of the driver preferably is less than or equal to the maximum outer diameter of the suture anchor. Driver **40** also features a slot **52** which is continuous with recess **50**.

The method of capsular plication proceeds using a 36-inch (91.4 cm) long #2 suture to plicate the capsulolabral complex. Both free ends of the suture are brought out an operative cannula. A spear with an included obturator is introduced through a skin incision or a clear cannula. The tip of the spear is positioned on bone and the obturator is removed.

A pilot hole is prepared in bone using either a punch or a drill depending on surgeon preference. With the manual punch, a mallet is used to advance the punch into bone until the punch handle meets the back of the spear and/or the shoulder on the distal part of the punch meets the bone surface. Alternatively, the drill can be attached with a Jacob chuck to a motorized drill and advanced until the stop on the drill bit meets the back of the spear.

After the pilot hole is created and the punch or drill is removed, the sterile-packaged implant **2** is opened to the sterile field using appropriate sterile technique. The implant is removed from the standard hand driver **20** and the suture is unloaded from the implant. A separate sterile packaged plication driver **40** is opened to the sterile field, One of the two legs of the plication suture is selected. This suture leg is the one on the medial side, or the one that passes under the tissue.

The selected suture leg is loaded through the implant eyelet. The implant **2** is positioned on plication driver **40** so that the open side of the eyelet **4** faces the open slot **52** on the driver. The suture leg will exit the slot **52** on the driver **40.** The implant with driver is pushed into the prepared pilot hole by hand. A mallet then is used to advance the implant into the hole. The implant is advanced until a second laser line **54** on the distal tip of the driver is flush with the bone surface and a laser line **56** on the proximal part of the implant driver shaft is flush with the back of the spear handle.

The implant driver handle is pulled straight off the implant and the spear is removed. Additional implants are inserted dependent upon the size of the soft tissue defect. Suture passing and knot tying are carried out in the preferred fashion.

Although the present invention has been described in relation to particular embodiments thereof, many other variations and modifications and other uses will become apparent to those skilled in the art.

## Claims

1. An insert-molded suture anchor (2) formed by placing a suture in a mold and molding a suture anchor body around the suture, the suture anchor comprising:
an anchor body (6) comprising a plurality of adjacent truncated cones (12), the anchor body terminating at its distal end (10) in a truncated cone with a blunt tip;
a round and tapered drive head (8) at the proximal end of the anchor body; and
a suture (4), insert-molded with the anchor body (6) in the form a suture loop, the suture loop extending outside the anchor body at the proximal end of the anchor to provide a suture eyelet.

2. An insert-molded suture anchor as recited in claim 1, further **characterized in that** the suture insert-molded within the anchor body extends through the entire length of the anchor.

3. An insert-molded suture anchor assembly comprising the insert molded suture anchor as recited in claim 1 in combination with a hand driver (20) having a cannulated shaft (22) with an open round recess (30) on an end of the shaft for receiving the round and tapered proximal end of the suture anchor (2).

4. A process of forming a suture anchor (2) comprising the steps of:
placing at least one piece of suture (4) in a mold;
molding a polymer around the suture by delivering an uncured polymer into the mold, and
causing the polymer to cure, to form an insert-molded suture anchor (2) having an anchor body (6) comprising a plurality of adjacent truncated cones (12), the anchor body terminating at its distal end (10) in a truncated cone with a blunt tip, a round and tapered drive head (8) at the proximal end of the anchor body, and a suture loop extending outside the anchor body at the proximal end of the anchor to provide a suture eyelet.

## Patentansprüche

1. Eingegossener Nahtmaterialanker (2), der gebildet wird, indem ein Nahtmaterial in eine Form gegeben wird und ein Nahtmaterialankerkörper um das Nahtmaterial herum geformt wird, wobei der Nahtmaterialanker Folgendes umfasst:
einen Ankerkörper (6), der eine Mehrzahl von aneinandergrenzenden Kegelstümpfen (12) umfasst, wobei der Ankerkörper an seinem distalen Ende (10) in einem Kegelstumpf mit einer stumpfen Spitze endet;
einen runden und konisch zulaufenden Antriebskopf (8) am proximalen Ende des Ankerkörpers; und
ein Nahtmaterial (4), das im Ankerkörper (6) in Form einer Nahtmaterialschleife eingegossen ist, wobei sich die Nahtmaterialschleife außerhalb des Ankerkörpers am proximalen Ende des Ankers erstreckt, um eine Nahtmaterialöse zu bilden.

2. Eingegossener Nahtmaterialanker, wie in Anspruch 1 angeführt, weiters **dadurch gekennzeichnet, dass** sich das im Ankerkörper eingegossene Nahtmaterial durch die gesamte Länge des Ankers erstreckt.

3. Eingegossene Nahtmaterialankeranordnung, umfassend den in Anspruch 1 angeführten eingegossenen Nahtmaterialanker in Kombination mit einem Handantrieb (20), der einen kanülierten Schaft (22) mit einer offenen runden Vertiefung (30) an einem Ende des Schafts zum Aufnehmen des runden und konisch zulaufenden proximalen Endes des Nahtmaterialankers (2) aufweist.

4. Verfahren zum Bilden eines Nahtmaterialankers (2), umfassend die folgenden Schritte:
das Legen von zumindest einem Stück Nahtmaterial (4) in eine Form;
das Gießen eines Polymers um das Nahtmaterial, indem ein ungehärtetes Polymer in die Form abgegeben wird, und das Bewirken, dass das Polymer aushärtet, um einen eingegossenen Nahtmaterialanker (2) zu bilden, der einen Ankerkörper (6) mit einer Mehrzahl von aneinandergrenzenden Kegelstümpfen (12), wobei der Ankerkörper an seinem distalen Ende (10) in einem Kegelstumpf mit einer stumpfen Spitze endet, umfasst, sowie einen runden und konisch zulaufenden Antriebskopf (8) am proximalen Ende des Ankerkörpers und eine Nahtmaterialschleife, die sich außerhalb des Ankerkörpers am proximalen Ende des Ankers erstreckt, um eine Nahtmaterialöse zu bilden.

## Revendications

1. Ancre (2) de suture moulée par insertion formée par placement d'une suture dans un moule et par moulage d'un corps d'ancre de suture autour de la suture, l'ancre de suture comprenant :
un corps (6) d'ancre comprenant une pluralité de cônes tronqués adjacents (12), le corps d'ancre se terminant, au niveau de son extrémité distale (10), en un cône tronqué à bout émoussé ;
une tête d'entraînement ronde et amincie (8) située à l'extrémité proximale du corps d'ancre ; et
une suture (4), moulée par insertion avec le corps (6) d'ancre sous la forme d'une boucle de suture, la boucle de suture s'étendant à l'extérieur du corps d'ancre au niveau de l'extrémité proximale de l'ancre pour fournir un oeillet de suture.

2. Ancre de suture moulée par insertion selon la revendication 1, **caractérisé en outre en ce que** la suture moulée par insertion à l'intérieur du corps d'ancre s'étend sur toute la longueur de l'ancre.

3. Ensemble d'ancre de suture moulée par insertion, comprenant une ancre de suture moulée par insertion selon la revendication 1 en combinaison avec un dispositif d'entraînement manuel (20) comportant une tige à canule (22) avec un évidement rond ouvert (30) sur une extrémité de la tige destiné à recevoir l'extrémité proximale ronde et amincie de l'ancre de suture (2).

4. Procédé de formation d'une ancre (2) de suture comprenant des étapes :
de placement d'au moins une pièce de suture (4) dans un moule ;
de moulage d'un polymère autour de la suture par délivrance d'un polymère non polymérisé dans le moule, et entraînant la polymérisation du polymère, pour former une ancre (2) de suture moulée par insertion comportant un corps (6) d'ancre comprenant une pluralité de cônes tronqués adjacents (12), le corps d'ancre se terminant, au niveau de son extrémité distale (10), en un cône tronqué à bout émoussé, une tête d'entraînement ronde et amincie (8) située au niveau de l'extrémité proximale du corps d'ancre, et une boucle de suture s'étendant à l'extérieur du corps d'ancre au niveau de l'extrémité proximale de l'ancre pour fournir un oeillet de suture.
